# EUROPEAN PATENT SPECIFICATION

(11) **EP 1 337 247 B1**
(45) Date of publication and mention of the grant of the patent: **16.08.2006**
(21) Application number: 01996378.4
(22) Date of filing: 20.11.2001
(51) Int. Cl.: A61K 31/197, A61K 31/195, A61K 31/4172, A61K 31/42, A61K 31/437, A61K 31/4535, A61P 5/24, A61P 25/24

(54) **Use of Gaboxadol for the treatment of premenstrual disorder, postnatal depression or postmenopausal related dysphoric disorder**
Verwendung von Gaboxadol zur Behandlung von premenstruelle Störung, postnatal Depression oder postmenopausal-bedingte-dysphorische Störung
Utilisation du Gaboxadol pour le traitement de troubles premenstruels, de la depression postnatale ou de troubles postménopausaux dysphoriques

(30) Priority: 20.11.2000 DK 200001743
(43) Date of publication of application: 27.08.2003
(73) Proprietor: H. LUNDBECK A/S, 2500 Valby-Copenhagen (DK)
(72) Inventor: EBERT, Bjarke, DK-3520 Farum (DK); HONGAARD ANDERSEN, Peter, DK-3500 Vaerlose (DK)
(74) Representative: Kjerrumgaard, Lars Bo
(86) International application number: PCT/DK2001/000773
(87) International publication number: WO 2002/040009

(56) References cited:
- WO-A1-00/05196
- WO-A1-01/42190
- WO-A1-93/05786
- WO-A1-96/16076
- WO-A1-98/38988
- WO-A1-99/37645
- US-A- 5 292 906
- US-A- 5 776 959

## Description

The invention provides the use of THIP (Gaboxadol) for the preparation of a medicament for the treatment of premenstrual disorder, posnatal depression or post-menopausal related dysphoric disorder.

### Background of the invention

Receptors for the major inhibitory neurotransmitter, gammaamino butyric acid (GABA), are divided into two main classes: GABA_{A} receptors which are members of the ligand gated ion channel superfamily; and the GABA_{B} receptors which are G-protein coupled receptors.

GABA_{A} receptors are formed as a pentameric assembly of different families of receptor subunits. The assembly, which in most receptors includes 2 α subunits, 2β subunits and a γ or δ subunit, determines the pharmacology of the functional receptor. The binding site for benzodiazepines is located at the interface between the α and γ subunit, whereas the binding site for GABA and other GABA_{A} agonists is located at the interface between the α and β subunit.
GABA_{A} receptor assemblies which do exist include, amongst many others, α₁β₂γ₂, α₁β_{2/3}γ₂, α₃Pγ_{2/3}, α₅β_{3γ2/2}, α₆βγ₂ α₆βδ, α₄βδ and α₄β₂γ₂. Subtypes containing the a₁ subunit are present in most brain regions and may contribute to the functional action of a number of benzodiazepines.

In a number of clinical conditions, hypoactivity of the inhibitory GABA system has been hypothesised as the underlying mechanism of the pathology in question. These conditions include epilepsy, anxiety, stress, sleep disorders and pain. However, although positive modulators of the GABA_{A} receptor complex, such as benzodiazepines, in a number of circumstances are very effective, there is a general consensus that unselective benzodiazepines produce so many side effects that compounds substituting for presently used drugs are needed (Costa and Guidotto *Trends Pharmacol. Sci.* **1996***, 17,* 192-200).

The α₄ containing receptors exist predominantly in the thalamic area (Sur et al. **1999**). Recent studies (Sassoè-Pognetto et al.. *J Camp Neurol* **2000**, *15,* 420: 481-98 ; Mody, 2000, Presentation at GABA2000 meeting July 23 to July 29.) have indicated that some of these receptors may be located extrasynaptically, making them a potentially very interesting drug target.
There are differences between benzodiazepines and GABA agonists. One is that benzodiazepines are inactive at α₄ and δ containing receptors, whereas GABA_{A} agonists will act irrespective of the subunit composition (e.g. Ebert et al. *Mol. Pharmacol.* **1997**, *52,* 1150-1156). Another, that the benzodiazepines react at a specific site at the GABA complex, thereby causing the GABA receptor to undergo an allosteric change which influences the efficacy of GABA in promoting chloride channel opening. The GABA receptor modulators exhibit considerable side-effects. In relation to disorders such as anxiety and pre-menstrual dysphoric disorder modulation of the thalamic areas may play a key role. In these areas a high abundance of α₄β₃δ/γ₂ containing receptors are found, making interaction with these receptors particularly interesting. With the large density of α₄ containing receptors located exstrasynaptically (Sur et al. *Mol. Pharmacol.* **1999**, *56*,110-115; Sassoè-Pognetto et al. *J Comp Neurol* **2000*****,** 15,*420: 481-98; Mody, 2000, Presentation at GABA2000 meeting July 23 to July 29) only a relatively low level of activation at the individual extrasynaptic receptors will sum up to a significant inhibition of the neurone, raising the possibility that highly functional selective compounds can be developed for these receptors.
The ovarian hormone progesterone and its metabolites have been demonstrated to have profound effects on brain excitability. The levels of progesterone and its metabolites vary with the phases of the menstrual cycle. It has been documented that progesterone and its metabolites decrease prior to the onset of menses. The monthly recurrence of certain physical symptoms prior to the onset of menses has also been well documented. These symptoms which have been associated with premenstrual syndrome (PMS) or premenstrual dysphoric disorder (PMDD) include stress, anxiety, and migraine headaches. Patients suffering from PMS have a monthly recurrence of symptoms that are present in premenses and absent in postmenses. In a similar fashion, a reduction in progesterone has also been temporally correlated with an increase in seizure frequency in female epileptics. A more direct correlation has been observed with a reduction in progesterone metabolites. In addition, for patients with primarily generalized petit mal epilepsy, the temporal incidences of seizures have been correlated with the incidence of the symptoms of PMS.
A syndrome also related to low progesterone levels is postnatal depression (PND). Immediately after delivery, progesterone levels decrease dramatically leading to the onset of PND. The symptoms of PND range from mild depression to psychosis requiring hospitalization. PND is also associated with severe anxiety and irritability. PND associated depression is amenable to treatment by classical antidepresants and women experiencing PND show an increased incidence of PMS.

Premenstrual dysphoric disorder (PMDD) is thought to be a consequence of the rapid drop in progesterone levels, and especially progesterone metabolites, which act as positive modulators of the GABAergic activity (Gallo and Smith, 1993 *Pharmacol. Biochem. Behav.* **46**, 897-904).
The effect of the neuroactive steroids with direct effect at the GABA_{A} receptor has been investigated. Although neurosteroids like alfaxalone and 3α-5α-dihydroxyprogesterone are interacting with all types of GABA receptors, data with α₄β₃δ containing receptors indicate that the potency and efficacy at the receptors are higher than at other types of GABA_{A} receptors. Neurosteroids have been developed for the treatment of PMDD and other indications, however side effects have resulted in discontinuation of most of these compounds. Further, a series of studies have shown that prolonged application of neurosteroids as hypnotics results in compensatory mechanisms which ultimately lead to dependence (Lancel et al. *J. Pharmacol. Exp. Ther.* **1997**, *282,* 1213-1218).

The present invention provides non-steroid compounds interacting directly with the recognition site at the GABA_{A} receptor as agonists or GABA uptake inhibitors or as enhancers of GABAergic activity, which all have beneficial effects in disease states relating to reduced neurostoroidal activation.
The diseases, including premenstrual syndrome, postnatal depression and post menopausal related dysphoric disorders, are significantly better treated with GABA_{A} agonists and GABA uptake inhibitors or enchancers of GABAergic activity than with benzodiazepines and neurosteroids which produce tolerance after short term treatment.

The present invention also provides specific non-allosteric GABA agonistic compounds useful for the treatment of the disorders relating to reduced neurosteroid activation. The compounds are known as useful in the treatment of other diseases and disorders.

### Detailed description of the invention

The invention provides the use of a non-steroid compound which increases GABA activity in the brain for the manufacture of a medicament for the treatment of disorders resulting from reduced neurosteroidal activation.

The invention provides the use of a compound as described above, wherein the non-steroid compound is THIP (Gaboxadol).

The invention also provides the use as described above, wherein the disease or disorder is resulting from fluctuations in the neurosteroid level.

In a preferred embodiment of the invention, the disease or disorder is premenstrual disorder, postnatal depression or postmenopausal related dysphoric disorder.

The invention also provides the use as above wherein the medicament is for administration as a unit dose.

In a preferred embodiment of the invention, the unit dose is containing the active ingredient in an amount from about 10 µg/kg to 10mg/kg body weight, preferably 25 µg/day/kg to 1.0 mg/day/kg, most preferably 0.1 mg/day/kg to 1.0 mg/day/kg body weight.

In a more preferred embodiment, the unit dose is containing the active ingredient in an amount from 0.1 mg/day/kg to 1.0 mg/day/kg body weight.

In an embodiment of the invention, the neurosteroid activation is caused by hormones.

In a preferred embodiment, this is progesterone. In another preferred embodiment of the invention, it is the metabolites of progesterone.

According to the invention, the compounds mentioned above may be used as the base of the compound or as a pharmaceutically acceptable acid addition salt thereof or as an anhydrate or hydrate of such salt.
According to the invention, the compounds mentioned above or a pharmaceutically acceptable salt thereof may be administered in any suitable way e.g. orally or parenterally, and it may be presented in any suitable form for such administration, e.g. in the form of tablets, capsules, powders, syrups or solutions or dispersions for injection. Preferably, and in accordance with the purpose of the present invention, the compound of the invention is administered in the form of a solid pharmaceutical entity, suitably as a tablet or a capsule or in the form of a suspension, solution or dispersion for injection.

Methods for the preparation of solid pharmaceutical preparations are well known in the art. Tablets may thus be prepared by mixing the active ingredients with ordinary adjuvants and/or diluents and subsequently compressing the mixture in a convenient tabletting machine. Examples of adjuvants or diluents comprise: corn starch, lactose, talcum, magnesium stearate, gelatine, lactose, gums and the like. Any other adjuvant or additive such as colourings, aroma, preservatives, etc. may also be used provided that they are compatible with the active ingredients.

The compound of the invention is most conveniently administered orally in unit dosage forms such as tablets or capsules, containing the active ingredient in an amount from about 10 µg/kg to 10mg/kg body weight, preferably 25 µg/day/kg to 1.0 mg/day/kg.

The effect of the compounds is tested in a pseudo pregnancy model wherein the progesterone level are fluctuating and especially the effect on the rapid decline is measured as described for example in Gallo et. al. *Pharmacol. Biochem. Behav.* **1993**, *46*, 897-904

### Results

### Rodent Model of PMS

The described model is a hormone withdrawal model of PMS in the rat, based on the prevailing hypothesis that dysphoric mood is predominantly associated with declining hormone levels (i.e., "hormone withdrawal") in women with PMS. Previous work (Nature 392: 926-930, 1998; J. Neurosci. 18: 5275-5284, 1998) has demonstrated that following a three week period of hormone exposure, withdrawal from elevated levels of the reproductive steroid progesterone 24 hrs after removal of a sc progesterone-filled implant produces a state of increased anxiety and lowered seizure threshold in female rats.

Further evidence that the α4 subunit is increased was provided by electrophysiology data demonstrating a striking insensitivity of hippocampal cells to the GABA-potentiating effect of a benzodiazepine (BDZ) lorazepam. (BDZ insensitivity is characteristic of α4-containing GABA receptors.)

### Detailed description of the Experiments:

### Animals

Female mice (Charles River) were housed in pairs under a 14 hour light and 10 hour dark cycle with food and water ad libitum. All animals were tested during the light portion of the circadian cycle. In female mice, estrous cycle stage was determined by microscopic examination of the vaginal lavage, as described previously (Smith, 1987) and by measures of vaginal impedance (Bartlewski, 1999 ; Bartos, 1977 ; Koto, 1987; Koto, 1987) throughout one entire cycle prior to testing. Only females in diestrous were used as subjects.

### Drugs and Hormone Administration

Progesterone (P) was administered rather than 3α,Sα-THP because it is known that elevated circulating levels of P, such as found during the estrous (or menstrual) cycle or after stress, (Persengiev, 1991; Barbaccia, 1996; Barbaccia, 1997; Korneyev, 1993; Wilson, 1997; Elman, 1997; Vallee, 2000; Purdy, 1991; Korneyev, 1993) are readily converted to 3a,5a-THP in the brain and result in 3a-5a THP levels sufficient to potentiate GABAergic inhibition (Schmidt, 1994 ; Smith, 1987 ; Seiki, 1975 ; Bitran, 1995; Karavolas, 1976; Vallee, 2000) and modulate GABAA-R subunit expression [Weiland, 1995].
Progesterone implants were made from silicone tubing (Nalgene Co, 1/16"i.d x 1/8" o.d.) was cut to size depending on the body weight of the animal (10 mm tubing per 100 g), filled with crystalline progesterone and sealed with silastic medical adhesive (Dow Corning). The sealed capsules were incubated overnight in a solution containing 1% gelatine and 0.9% saline in a water bath (37 °C) with gentle shaking overnight. Sham implants are empty sealed tubes of the same dimensions. Rats were then anesthestized with 2% halothane (2-bromo-2-chloro-1,1,1-trifluroethane) in oxygen and the capsules implanted subcutaneously in the abdomen. Removal of the implants also occurred under the same regime of halothane anesthesia, and implanted s.c. under anesthesia in the abdominal area of the rat (Smith, 1998; Moran, 1998) for 21 days. This method has been shown to result in CNS levels of 3a,5a-THP in the high physiological range (6-12 ng/gm hippocampal tissue) in association with increased circulating levels of P (40-50 ng/ml plasma, approximately 130-160 nM) (Smith, 1998).
Control animals were implanted exactly the same way with empty (sham) silicone capsules. Animals were either sacrificed or tested 24 hrs after removal of the implant (P withdrawal).

On the day of testing, animals were injected with either THIP (1.25 mg/kg) or saline and tested 40 minutes after the injection.

### Behavioral Testing

Mice were tested on the plus maze, elevated 50 cm above the floor, in a room with low, indirect incandescent lighting and low noise levels. The plus maze consists of 2 enclosed arms (50 x 10 x 40 cm) and 2 open arms (50 x 10 cm) and is explained in detail in (Pellow, 1985). The open arms had a small rail outside the first half of the open arm as described in (Fernandes, 1996).
The floor of all four arms was marked with grid lines every 25 cm. On the day of testing, each mouse was placed in the testing room for 30-40 minutes prior to testing in order to acclimatise to the situations. At the time of testing, each animal was tested for 10 minutes after exiting a start box in the centre platform of the plus maze. To be considered as an entry into any arm, the mouse must pass the line of the open platform with all four paws. The duration (in seconds) of time spent in the open arm was recorded from the time of entry into the open arm. Decreased time spent in the open arm generally indicates higher levels of anxiety (Pellow, 1985). Other behavioural measures recorded included the duration of time spent (in seconds) beyond the rail. The amount of time that subjects spend in the open portion of the plus maze in the absence of rails is considered to be more sensitive to anxiolytic agents (i.e. agents that would increase the amount of time spent in the open arm) than the amount of time spent in the open arms with rails (Fernandes, 1996). In order to measure general locomotor activity, the number of total grid crosses was counted. Lastly, the duration of time (in sec) spent grooming was also scored.
The experimenter was blind to all conditions, and animals were tested in a randomised block design.

### Statistical analysis

Data from the plus maze were analysed in a 2-way ANOVA (implant condition x injection condition) followed by a post-hoc ANOVA and post hoc t-test. As illustrated in table 1, PWD mice spend significantly less time in the open arm than the control animals.

**Table 1:**

| **Means Table for Time Open Arm** | | | | |
|---|---|---|---|---|
| **Effect: Sex/Cond** | | | | |
| **Row exclusion: stvw PWD +M F/M D** | | | | |
| | Count | Mean | Std. Dev. | Std. Err. |
| (F) C | 14 | 79.629 | 59.231 | 15.830 |
| (F) PWD | 13 | 20.968 | 24.292 | 6.737 |
| (F) C THIP (1.25) | 3 | 38.377 | 48.816 | 28.184 |
| (F) PWD THIP (1.25) | 3 | 157.023 | 36.838 | 21.268 |

Furthermore, THIP at a dose of 1,25 mg/kg completely reversed the PWD effect. Similar results were obtained when the number of crossings (Table 2)

**Table 2:**

| **Means Table for Grid Cross** | | | | |
|---|---|---|---|---|
| **Effect: Sex/Cond** | | | | |
| **Row exclusion: stvw PWD +M F/M D** | | | | |
| | Count | Mean | Std. Dev. | Std. Err. |
| (F) C | 14 | 43.643 | 18.270 | 4.883 |
| (F) PWD | 13 | 33.308 | 18.531 | 5.140 |
| (F) C THIP (1.25) | 3 | 52.000 | 18.028 | 10.408 |
| (F) PWD THIP (1.25) | 3 | 83.333 | 16.166 | 9.333 |

The time spend outside the rail was determined (Table 3).

**Table 3:**

| **Means Table for Time Outside Rail** | | | | |
|---|---|---|---|---|
| **Effect: Sex/Cond** | | | | |
| **Row exclusion: stvw PWD +M F/M D** | | | | |
| | Count | Mean | Std. Dev. | Std. Err. |
| (F) C | 14 | 6.795 | 7.041 | 1.882 |
| (F) PWD | 13 | 2.077 | 4.699 | 1.303 |
| (F) C THIP (1.25) | 3 | 10.060 | 17.424 | 10.060 |
| (F) PWD THIP (1.25) | 3 | 29.503 | 6.699 | 3.868 |

As seen from the results of the animal models THIP was able to counteract the PWD completely.

## Claims

1. The use of THIP (Graboxadol) for the manufacture of a medicament for the treatment of a disease or disorder which is selected from premenstrual disorder, postnatal depression or postmenopausal related dysphoric disorder.

2. Use according to claim 1 wherein the medicament is for administration as a unit dose.

3. Use according to claim 2 wherein the unit dosage form contains the active ingredient in an amount from about 10 µg/kg to 10mg/kg body weight, preferably 25 µg/day/kg to 1.0 mg/day/kg.

## Patentansprüche

1. Verwendung von THIP (Gaboxadol) zur Herstellung eines Medikaments zur Behandlung einer Krankheit oder Störung, die aus prämenstrueller Störung, postnataler Depression oder postmenopausal-bezogener dysphorischer Störung ausgewählt ist.

2. Verwendung gemäß Anspruch 1, worin das Medikament für die Verabreichung als Einzeldosis ist.

3. Verwendung gemäß Anspruch 2, worin die Einheitsarzneiform den aktiven Bestandteil in einer Menge von ca. 10 µg/kg bis 10 mg/kg Körpergewicht enthält, bevorzugt 25 µg/Tag/kg bis 1,0 mg/Tag/kg.

## Revendications

1. Utilisation de la THIP (Gaboxadol) pour la fabrication d'un médicament pour le traitement d'une maladie ou d'un trouble qui est choisi(e) parmi le trouble prémenstruel, la dépression postnatale ou le trouble dysphorique associé à la post-ménopause.

2. Utilisation selon la revendication 1 dans laquelle le médicament est destiné à une administration en tant que dose unitaire.

3. Utilisation selon la revendication 2 dans laquelle la forme de dose unitaire contient l'ingrédient actif dans une quantité d'environ 10 µg/kg à 10 mg/kg de poids corporel, préférablement 25 µg/jour/kg à 1,0 mg/jour/kg.
